(19) Europäisches Patentamt
European Patent Office
Office européen des brevets

(11) Publication number : 0 428 343 B1

# (12) EUROPEAN PATENT SPECIFICATION

(45) Date of publication of patent specification : 04.05.94 Bulletin 94/18

(51) Int. Cl.[5] : C07C 45/54, C07C 49/597

(21) Application number : 90312269.5

(22) Date of filing : 09.11.90

## (54) A process for producing an alpha,beta-unsaturated carbonyl compound.

(30) Priority : 11.11.89 JP 293697/89

(43) Date of publication of application : 22.05.91 Bulletin 91/21

(45) Publication of the grant of the patent : 04.05.94 Bulletin 94/18

(84) Designated Contracting States : CH DE FR GB LI

(56) References cited :
US-A- 4 575 570
PATENT ABSTRACTS OF JAPAN, unexamined applications, C section, vol. 9, no. 160, July 4, 1985 THE PATENT OFFICE JAPANESE GOVERNMENT page 148 C 289
PATENT ABSTRACTS OF JAPAN, unexamined applications, C section, vol. 9, no. 53, June 27, 1985 THE PATENT OFFICE JAPANESE GOVERNMENT page 111 C 288

(56) References cited :
PATENT ABSTRACTS OF JAPAN, unexamined applications, C section, vol. 9, no. 153, June 27, 1985 THE PATENT OFFICE JAPANESE GOVERNMENT page 110 C 288
PATENT ABSTRACTS OF JAPAN, unexamined applications, C section, vol. 8, no. 178, August 16, 1984 THE PATENT OFFICE JAPANESE GOVERNMENT page 27 C 238
PATENT ABSTRACTS OF JAPAN, unexamined applications, C section, vol. 9, no. 45, February 26, 1985 THE PATENT OFFICE JAPANESE GOVERNMENT page 14 C 268

(73) Proprietor : NIPPON ZEON CO., LTD.
6-1, Marunouchi 2-chome
Chiyoda-ku Tokyo 100 (JP)

(72) Inventor : Watanabe, Kiyoshi
308, Gumyojicho
Minami-ku, Yokohama-shi (JP)
Inventor : Matsuoka, Rikitaro
873, Futoocho
Kohoku-ku, Yokohama-shi (JP)

(74) Representative : Stuart, Ian Alexander et al
MEWBURN ELLIS York House 23 Kingsway
London WC2B 6HP (GB)

EP 0 428 343 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a process for producing an α,β-unsaturated carbonyl compound. More particularly, it relates to a process for producing an α,β-unsaturated carbonyl compound useful for production of chemical substances such as medicines, agricultural chemicals, perfumes, by the reaction of an alkenyl ester and an allyl type carbonic ester.

α,β-unsaturated carbonyl compounds such as 2-cyclopentenone, 2-cyclohexenone, 2-cyclododecenone are very useful chemical substances in the fields of medicines, agricultural chemicals, perfumes.

As a process disclosed in Patent Abstract of Japan Kokai No. 60-36435 for producing such an α,β-unsaturated carbonyl compound, there has been known, for example, a process which comprises reacting, as illustrated by the formula shown below, an alkenyl ester such as 1-cyclopentenyl acetate with an allyl type carbonic ester such as allyl methyl carbonate in the presence of a platinum group metal compound catalyst.

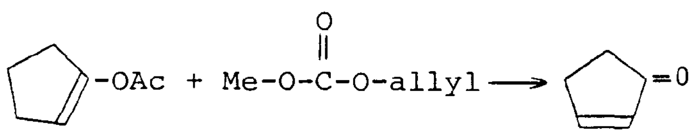

This process uses easily available compounds as starting materials and permits relatively efficient production of an α,β-unsaturated carbonyl compound. It, however, requires selective separation and purification of the reaction product, i.e., the α,β-unsaturated carbonyl compound because the actual reaction mixture contains the starting allyl type carbonic ester and a by-product allyl acetate in addition to the α,β-unsaturated carbonyl compound.

For reducing the amount of the residual starting allyl type carbonic ester in the reaction mixture, the reduction of the amount of the allyl type carbonic ester used is thought of. But, the reduction of the allyl type carbonic ester is substantially impossible because for producing the α,β-unsaturated carbonyl compound, it is indispensable to use the allyl type carbonic ester in an equivalent amount to or larger than the amount of the alkenyl ester. Therefore, the remaining of the allyl type carbonic ester in the reaction mixture is unavoidable.

On the other hand, as a method for the separation and purification of the α,β-unsaturated carbonyl compound from the reaction mixture, purification by distillation is thought of at first. However, when the boiling point of the allyl type carbonic ester which remains as unreacted starting compound is close to that of the desired product, i.e., the α,β-unsaturated carbonyl compound, they cannot be separated from each other by distillation. Therefore, there is required a method by which the α,β-unsaturated carbonyl compound can be separated efficiently in high purity by a means other than distillation.

As information concerning a method for this separation, it has been reported that allyl carbonate can be decomposed by reacting triphenylphosphine and palladium acetate with allyl carbonate in the molar ratio of triphenylphosphine/palladium acetate = 5 with heating (Tetrahedron Letters, 12, 3591 (1981)).

However, close investigation by the present inventors proved that when the above information was applied to decomposition of the by-product allyl acetate, allyl acetate could not be decomposed by such a method.

In addition, the by-product allyl acetate can be decomposed by hydrolysis with a strong acid or a strong alkali or by heating at a high temperature, but when such a decomposition method is applied to a reaction system for producing the α,β-unsaturated carbonyl compound, decomposition of the desired α,β-unsaturated carbonyl compound is inavoidable.

Patent Abstracts of Japan, unexamined applications, C Section, Vol. 8, no. 178 16 August 1984 page 27C 238 Kokai No. 59-73536 reports on a process for producing α,β-unsaturated ketone by contacting a carbonic acid diester with catalyst composed of a platinum-group metallic compound and an α,ω-alkylene di(di-substituted) phosphine. It is described that the molar ratio of α,ω-alkylene di(di-substituted) phosphine (B) per 1 mole of a platinum-group metallic compound (A) is preferably 0.7 - 1.5 mole. The staring material disclosed in this reference is a carbonic acid diester which is different from the allyl-type carbonic ester or alkenyl ester of the present invention.

Accordingly, it has been very difficult to separate and purify the α,β-unsaturated carbonyl compound efficiently in high purity from the reaction mixture obtained by the reaction of the alkenyl ester with the allyl type carbonic ester, by a heretofore known method.

The present invention was made in consideration of such conditions and is intended to ameliorate the defects in the prior art and/or other problems and provide a novel process for producing an α,β-unsaturated carbonyl compound which makes it possible to obtain the α,β-unsaturated carbonyl compound efficiently in high purity from a reaction mixture.

That is, the present invention provides a process for producing an α,β-unsaturated carbonyl compound represented by the general formula (II):

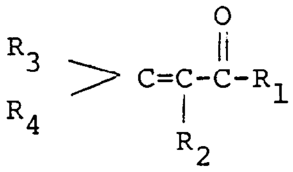 (II)

wherein $R_1$, $R_2$, $R_3$ and $R_4$ are independently hydrogen atoms or hydrocarbon residues, and $R_1$, $R_2$, $R_3$ and $R_4$ may be linear or may form one or more rings when taken together in arbitrary combinations

which comprises subjecting an alkenyl ester represented by the general formula (I):

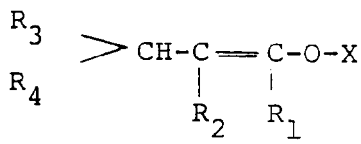 (I)

wherein $R_1$, $R_2$, $R_3$ and $R_4$ have the same meanings as those defined above, and X is an acyl group

and a carbonic ester having at least one allyl or its allied residue and represented by the general formula (III):

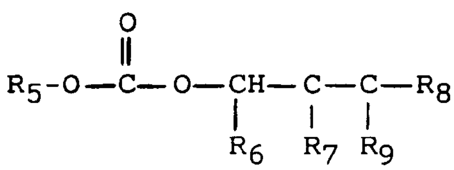

wherein $R_5$ is a hydrocarbon residue and $R_6$, $R_7$, $R_8$ and $R_9$ are independently hydrogen atoms or hydrocarbon residues

to catalytic reaction in the presence of a platinum group metal compound catalyst, and then adding a phosphorus-containing compound, followed by heating and wherein the total amount of the phosphorous-containing compound used in the catalytic reaction and the reaction with heating is at least 3 moles per mole of the platinum group metal compound catalyst.

In the production process of the present invention, easily available compounds can be used as starting materials as in conventional processes. As the starting alkenyl ester, there is used a compound of the general formula (I):

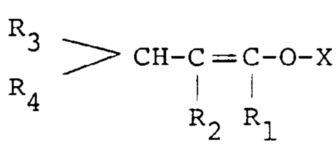 (I)

In the formula (I), $R_1$, $R_2$, $R_3$ and $R_4$ are independently hydrogen atoms or hydrocarbon residues. As the hydrocarbon residues, there can be exemplified by alkyl groups such as methyl, ethyl, propyl, and pentyl; phenyl group; and trityl group. $R_1$, $R_2$, $R_3$ and $R_4$ may be taken together in arbitrary combinations to form one or more rings. For example, $R_1$ may be bonded to $R_2$, $R_3$ or $R_4$ to form a ring such as a cyclopentane, cyclopentene, cyclohexane, cyclohexene, cyclododecane, cyclododecene ring or the like. X in the formula (I) is an acyl group.

Specific examples of the alkenyl ester of the formula (I) described above are 1-cyclopentenyl acetate, 1-cyclohexenyl acetate, 6-methyl-1-cyclohexenyl acetate, 6-ethyl-1-cyclohexenyl acetate, 1-cycloheptenyl acetate, 1-cyclododecenyl acetate, 1-cyclohexenyl propionate, 1-cyclohexenyl butyrate, 1-cyclohexenyl benzoate, 1-phenyl-1-butenyl acetate, 1-propenyl acetate, 1-hexenyl acetate, 3-methyl-1-propenyl acetate, 3-phenyl-1-propenyl acetate. Of these, cycloalkenyl esters are preferable. In particular, cycloalkenyl esters having a 5- to 7-membered ring or a 12-membered ring are preferable. As the alkenyl esters exemplified above, those synthesized by conventional methods can be used. For example, 1-cyclopentenyl acetate can easily be obtained by reacting cyclopentanone with isopropenyl acetate in the presence of an acid or by reacting cyclopentanone with acetic anhydride.

The allyl type carbonic ester, i.e., another starting material in the production process of the present invention, is a carbonic ester having at least one allyl or its allied residue and can be represented by the formula (III).

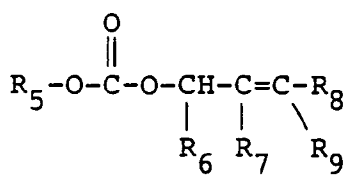

(III)

wherein $R_5$ is a hydrocarbon residue, and $R_6$, $R_7$, $R_5$ and $R_9$ are independently hydrogen atoms or hydrocarbon residues.

Specific examples of such an allyl type carbonic ester are allyl methyl carbonate, allyl propyl carbonate, allyl butyl carbonate, allyl pentyl carbonate, crotyl methyl carbonate, ethyl methallyl carbonate, diallyl carbonate. Of these, there are preferably used allyl esters, crotyl esters and methallyl esters, in which $R_5$ is a lower alkyl group having 4 or less carbon atoms.

As to the proportions of the amount of alkenyl ester and the allyl type carbonic ester, the allyl type carbonic ester is usually used in an amount of 0.8 to 5 moles, preferably 1 to 3 moles, per mole of the alkenyl ester.

In the reaction of the alkenyl ester with the allyl type carbonic ester, a platinum group metal compound catalyst is used. As the platinum group metal compound catalyst, there may be used either a platinum metal compound per se or a catalyst composed of a platinum group metal compound and a ligand.

As the platinum group metal compound, there can be used, for example, various inorganic acid salts, organic acid salts or complexes of palladium, platinum, rhodium, iridium or ruthenium. More specific examples thereof are tris(dibenzylideneacetone)dipalladium (0), tris(tribenzylideneacetylacetone)tripalladium (0), palladium acetate, palladium propionate, palladium butyrate, palladium benzoate, palladium acetylacetonate, palladium nitrate, palladium sulfate, palladium chloride, platinum [II] acetate, platinum acetylacetonate. These platinum group metal compounds may be used singly or in combination of two or more thereof.

In these platinum group metal compounds, palladium is particularly preferable as platinum group metal element from the viewpoint of reactivity. Zero-valent olefin complexes or divalent organic compounds are also preferable examples of the platinum group metal compound.

In the case where an inorganic strong acid salt is used as the platinum group metal compound catalyst, it is preferable to place together therewith potassium acetate, sodium alkoxides, tertiary amines or the like.

As the ligand, there can be used monodenate or multidentate electron donor compound having an element of Group V of the periodic table, i.e., nitrogen, phosphorus, arsenic or antimony.

Specific examples of the ligand are nitrogen-containing compounds such as pyridine, quinoline, trimethylamine, triethylamine, tributylamine, α,α'-dipyridine, 1,10-phenanthroline, N,N,N',N'-tetramethylethylenediamine, etc.; phosphorus-containing compounds such as triethylphosphine, tri-n-butylphosphine, tri-n-dodecylphosphine, triphenylphosphine, tri-o-tolylphosphine, tri-p-biphenylphosphine, tri-o-methoxyphenylphosphinel phenyldiphenoxyphosphine, triethyl phosphite, tri-n-butyl phosphite, tri-n-hexyl phosphite, triphenyl phosphite, tri-o-tolyl phosphite, triphenyl thiophosphite, α,β-ethylenedi(diphenyl)phosphine, α,β-ethylenedi(dibutyl)phosphine, α,γ-propylenedi(diphenyl)phosphine; arsenic-containing compounds such as triethylarsine, tributylarsine, triphenylarsine; and antimony-containing compounds such as tripropylstibine, triphenylstibine. Of these, the nitrogen-containing compounds and the phosphorus-containing compounds are preferable from the viewpoint of the activity, selectivity and economical benefit of reaction.

Such a ligand is, of course, not always indispensable as a constituent of the catalyst. But, the stability of the catalyst can be greatly improved by using the ligand in a properly adjusted amount. The amount of the ligand used is usually 2.5 moles or less, preferably about 0.1 to about 2 moles, per mole of the platinum group metal compound.

When the ligand is used, the platinum group metal compound and the ligand may be previously reacted with each other, or they may be added to a reaction system separately to prepare the catalyst in the reaction system.

The amount of the platinum group metal compound catalyst used is properly determined depending on the kinds of the alkenyl ester and the catalyst. It is usually in such a proportion that the amount of the platinum

group metal compound is 0.01 to 10 moles, preferably 0.1 to 5 moles, per 100 moles of the alkenyl ester.

In the present invention, a diluent can be placed in a reaction system for subjecting the alkenyl ester and the allyl type carbonic ester to catalytic reaction in the presence of the platinum group metal compound catalyst.

As the diluent, there can be exemplified nitriles such as acetonitrile, propionitrile, benzonitrile; amides such as dimethylformamide, diethylformamide, dimethylacetamide, dimethylpropioamide, N-methylpyrrolidone; ethers such as tetrahydrofuran, dioxane, dibutyl ether, ethylene glycol dimethyl ethers; ketones such as acetone, methyl ethyl ketone, methyl isobutyl ketone, cyclohexanone; esters such as methyl acetate, ethyl acetate, propyl acetate, ethyl propionate; alcohols such as ethanol, propanol, tert-butanol, ethylene glycol, diethylene glycol, diethylene glycol monoethyl ether; sulfoxides such as dimethyl sulfoxide, diethyl sulfoxide; alkanes such as n-hexane, cyclohexane; and aromatic hydrocarbons such as benzene, toluene, xylene. Of these, there are usually used aprotic polar solvents, in particular, the nitriles, the amides, the ethers, the ketones and the esters.

Such a diluent can improve the activity and selectivity of the reaction and the stability of the catalyst.

The amount of the diluent used depends on the kinds of starting materials and the catalyst. Usually, the diluent is used preferably in such a proportion that the total concentration of the alkenyl ester and the allyl type carbonic ester becomes 1 to 50% by weight.

The reaction temperature is usually 50°C or higher, preferably 55°C to 150°C. The reaction time is usually 10 minutes to 20 hours.

The production process of the present invention is characterized also in that after the reaction is carried out in the manner described above, a phosphorus-containing compound is added to the reaction mixture followed by heating. By the addition of a phosphorus-containing compound followed by heating, the uncreacted allyl type carbonic ester and the by-product which remain in the reaction mixture can be decomposed and removed at the same time. Accordingly, a desired α,β-unsaturated carbonyl compound alone can easily be separated and purified.

As the phosphorus-containing compound added in this case, there can be used, for example, the phosphorus-containing compounds exemplified as the ligand of the above-mentioned platinum group metal compound catalyst.

As to the amount of the phosphorus-containing compound added, the total amount of the phosphorus-containing compound used in the previous catalytic reaction and the subsequent reaction with heating is such that the molar ratio of the phosphorus-containing compound to the platinum group metal compound is at least 3:1. When the phosphorus-containing compound is added in such an amount, not only the unreacted allyl type carbonic ester but also the by-product can completely decomposed and removed. When the phosphorus-containing compound is present as the ligand of the catalyst in the previous catalytic reaction, the total molar ratio of the phosphorus-containing compound to the platinum group metal compound is adjusted by adjusting the amount of the phosphorus-containing compound added in the subsequent reaction with heating. The phosphorus-containing compound may be used in an amount of much larger than 3 moles per mole of the platinum group metal compound, but too large an amount thereof is not economical and therefore the amount thereof is preferably 10 moles or less. However, when the phosphorus-containing compound is used in an amount of 2 moles or more in the previous catalytic reaction, the reaction does not proceed depending on the kinds of the starting materials, and hence care should be taken.

Reaction conditions:

Temperature: usually 50°C or higher, preferably 55°C to 150°C.
Time: 30 minutes to 10 hours

Since the unreacted starting compound and the by-product are effectively decomposed by the reaction with heating described above, a desired α,β-unsaturated carbonyl compound can be separated and purified efficiently in high purity by distillation.

The present invention is more specifically illustrated with the following examples.

Example 1

20 mmole of 1-cyclopentenyl acetate and 24 mmoles of allyl methyl carbonate were subjected to catalytic reaction in the presence of 0.2 mmole of palladium acetate ($Pd(OAc)_2$) in 10 ml of acetonitrile at reflux temperature of the solvent for 2 hours. The reaction mixture was analyzed for its components by gas chromatography. Consequently, it was confirmed that the reaction mixture contained 9.28% by area of allyl methyl carbonate and 3.88% by area of allyl acetate in addition to 2-cyclopentenone.

Then, 0.6 mmole of triphenylphosphine ($PPh_3$) was added to the reaction system and the resulting mixture

was heated at the reflux temperature of the solvent for 4 hours.

The reaction mixture thus obtained was analyzed for its components as above. Consequently, neither allyl methyl carbonate nor allyl acetate was detected.

The results described above are summarized in Table 1.

Subsequently, the reaction mixture was distilled under conditions of 40 mmHg and 67°C to give 2-cyclopentenone in 93% yield.

Thus, it was confirmed that the subsequent reaction in the present invention, i.e., the heating after addition of the phosphorus-containing compound, is effective in decomposing and removing the unreacted starting material and the by-product.

Example 2

The process of Example 1 is repeated except for adding 0.2 mmole of triphenylphosphine in the previous catalytic reaction, and changing the amount of triphenylphosphine added in the subsequent reaction with heating and the period of the subsequent reaction with heating to 0.4 mmole and 3 hours, respectively. The results of analysis of the reaction mixtures are also shown in Table 1.

Also in the present example, neither the unreacted starting material nor the by-product was detected in the reaction mixture obtained by the subsequent reaction with heating, and 2-cyclopentenone was obtained in 94% yield.

Comparative Example 1

The process of Example 1 was repeated except that in the previous catalytic reaction, the amount of triphenylphosphine added and the reaction time were changed to 0.2 mmole and 3.5 hours, respectively, and that in the subsequent reaction with heating, the amount of triphenylphosphine added and the reaction time were changed to 0.2 mmole and 5 hours, respectively. The results of analysis of the reaction mixtures are shown in Table 1.

It was confirmed that when the amount of the phosphorus-containing compound added is small, allyl acetate remains without decomposition.

Referential Example

In a reactor were placed 10 mmoles of allyl acetate, 0.1 mmole of palladium acetate, 0.5 mmole of triphenylphosphine and 10 ml of acetonitrile, and the reaction was carried out at a temperature of 50°C for 2 hours.

The reaction did not proceed, that is, allyl acetate was not decomposed.

Table 1

| | Previous catalytic reaction | | | | Subsequent reaction | | | |
|---|---|---|---|---|---|---|---|---|
| | $pph_3$/ $Pd(OAc)_2$ molar ratio | Reaction time (hr) | Allyl methyl carbonate (%)* | Allyl acetate (%)* | $pph_3$/ $Pd(OAc)_2$ molar ratio | Reaction time (hr) | Allyl methyl carbonate (%)* | Allyl acetate (%)* |
| Example 1 | 0 | 2 | 9.28 | 3.88 | 3 | 4 | 0 | 0 |
| Example 2 | 1 | 2 | 11.5 | 2.91 | 2 | 3 | 0 | 0 |
| Comparative Example 1 | 1 | 3.5 | 10.7 | 3.88 | 1 | 5 | 0 | 1.13 |

Note: *: expressed in terms of a percentage by area anlyzed by gas chromatography.

Example 3

The procedure of Example 1 was repeated except that 0.4 mmole of pyridine was added and the reaction time was changed to 10 hours in the previous catalytic reaction.

The reaction mixture thus obtained was analyzed for its components as above. Consequently, neither allyl methyl carbonate nor allyl acetate was detected; but it was confirmed that 2-cyclopentenone was obtained in 90% yield.

The production process of the present invention makes it possible to decompose and remove the unreacted starting material and the by-product in a reaction mixture effectively by the subsequent reaction, and therefore it facilitates separation and purification of an α,β-unsaturated carbonyl compound. This process can give an α,β-unsaturated carbonyl compound in high yield and purity.

## Claims

1. A process for producing an α,β-unsaturated carbonyl compound represented by the general formula (II):

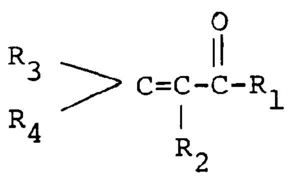

(II)

wherein $R_1$, $R_2$, $R_3$ and $R_4$ are independently hydrogen atoms or hydrocarbon residues, and $R_1$, $R_2$, $R_3$ and $R_4$ may be linear or may form one or more rings when taken together in arbitrary combinations which comprises subjecting an alkenyl ester represented by the general formula (I):

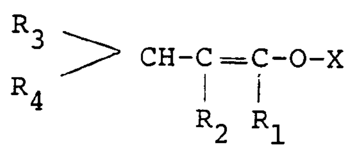

(I)

wherein $R_1$, $R_2$, $R_3$ and $R_4$ have the same meanings as those defined above, and X is an acyl group and a carbonic ester having at least one allyl or its allied residue and represented by the general formula (III):

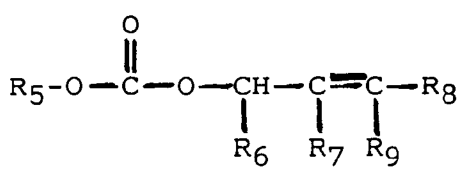

wherein $R_5$ is a hydrocarbon residue and $R_6$, $R_7$, $R_8$ and $R_9$ are independently hydrogen atoms or hydrocarbon residues

to catalytic reaction in the presence of a platinum group metal compound catalyst, and then adding a phosphorus containing compound followed by heating and wherein the total amount of the phosphorous-containing compound used in the catalytic reaction and the reaction with heating is at least 3 moles per mole of the platinum group metal compound catalyst.

2. The process according to Claim 1, wherein $R_1$, $R_2$, $R_3$ and $R_4$, which may be the same or different, are hydrogen atoms, alkyl groups, alkylene groups or aryl groups.

3. The process according to Claim 1, wherein the alkenyl ester is a cycloalkenyl ester.

4. The process according to Claim 3, wherein the cycloalkene is a 5- to 7-membered ring olefin or a 12-membered ring olefin.

5. The process according to Claim 4, wherein the cycloalkene is a 5-membered ring olefin.

6. The process according to Claim 1, wherein the allyl type carbonic ester is a lower alkyl ester of allyl carbonate, crotyl carbonate or methallyl carbonate.

7. The process according to Claim 1, wherein the allyl type carbonic ester is used in amount of 0.8 to 5 moles per mole of the alkenyl ester.

8. The process according to Claim 7, wherein the allyl type carbonic ester is used in amount of 1 to 5 moles per mole of the alkenyl ester.

9. The process according to Claim 1, wherein the platinum group metal compound catalyst is a platinum group metal compound or a catalyst composed of a platinum group metal compound and a ligand.

10. The process according to Claim 9, wherein the platinum group metal compound is a salt or a complex of a platinum group metal selected from palladium, platinum, rhodium, iridium or ruthenium.

11. The process according to Claim 10, wherein the platinum group metal is palladium.

12. The process according to Claim 11, wherein the palladium is in the form of a zerovalent olefin complex or a divalent organic compound.

13. The process according to Claim 9, wherein the ligand is a monodentate or multidentate electron donor compound having, as a ligand atom, an element of Group V of the periodic table.

14. The process according to Claim 13, wherein the ligand atom is one selected from nitrogen, phosphorus, arsenic and antimony.

15. The process according to Claim 14, wherein the ligand is a nitrogen-containing compound or a phosphorus-containing compound.

16. The process according to Claim 9, wherein the ligand is used in amount of 2.5 moles or less per mole of the platinum group metal compound.

17. The process according to Claim 16, wherein the ligand is used in amount of about 2 moles or less per mole of the platinum group metal compound.

18. The process according to Claim 1, wherein the platinum group metal compound catalyst is used in amount selected from 0.01 to 10 moles per 100 moles of the alkenyl ester.

19. The process according to Claim 1, wherein the catalytic reaction is carried out in the presence of a diluent.

20. The process according to Claim 19, wherein the diluent is one selected from nitriles, amides, ethers, ketone, esters, alcohols, sulfoxides and hydrocarbons.

21. The process according to Claim 19, wherein the diluent is an aprotic polar solvent.

22. The process according to Claim 1, wherein the diluent is used in such an amount that the total concentration of the alkenyl ester and the allyl type carbonyl ester becomes 1 to 50% by weight.

23. The process according to Claim 1, wherein in the catalytic reaction, the reaction temperature is 50°C or higher and the reaction time is 10 minutes to 20 hours.

24. the process according to Claim 1, wherein the phosphorus-containing compound is a monodentate or multidentate electron donor compound containing phosphorus.

25. The process according to Claim 24, wherein the phosphorus-containing compound is a phosphine or a phosphite.

26. The process according to Claim 25, wherein the phosphine is a trialkylphosphine or triphenylphosphine.

27. The process according to Claim 1, wherein in the reaction with heating, the reaction temperature is 50°C or higher and the reaction time is 30 minutes to 10 hours.

**Patentansprüche**

1. Verfahren zur Herstellung einer α,β-ungesättigten Carbonylverbindung der allgemeinen Formel (II)

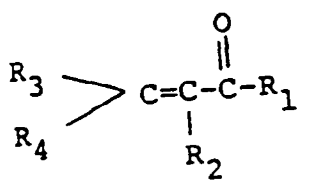

(II)

in der $R_1$, $R_2$, $R_3$ und $R_4$ unabhängig Wasserstoffatome oder Kohlenwasserstoffreste sind und $R_1$, $R_2$, $R_3$ und $R_4$ linear sein können oder in beliebigen Kombinationen miteinander einen oder mehrere Ringe bilden können, umfassend, das Durchführen einer katalytischen Umsetzung mit einem Alkenylester der allgemeinen Formel (I)

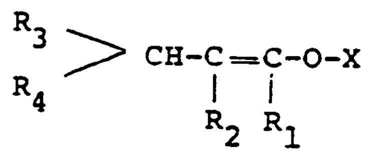

(I)

in der $R_1$, $R_2$, $R_3$ und $R_4$ die vorstehend definierte Bedeutung haben und X ein Acylrest ist und einem Kohlensäureester der mindestens einen Allyl- oder damit verwandten Rest der allgemeinen Formel (III)

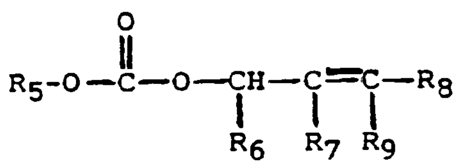

(III)

in der $R_5$ ein Kohlenwasserstoffrest ist und $R_6$, $R_7$, $R_8$ und $R_9$ unabhängig Wasserstoffatome oder Kohlenwasserstoffreste sind, in Gegenwart einer Verbindung eines Platinmetalls als Katalysator, und dann die Zugabe einer phosphorhaltigen Verbindung zusetzt, und nachfolgendes Erhitzen, wobei die Gesamtmenge der phosphorhaltigen Verbindung, die in der katalytischen Umsetzung und der Umsetzung unter Erhitzen eingesetzt wird, mindestens 3 Mole pro Mol der Verbindung eines Platinmetalls als Katalysator beträgt.

2. Verfahren nach Anspruch 1, in dem $R_1$, $R_2$, $R_3$ und $R_4$, die gleich oder verschieden sein können, Wasserstoffatome, Alkyl-, Alkylen- oder Arylreste sind.

3. Verfahren nach Anspruch 1, in dem der Alkenylester ein Cycloalkenylester ist.

4. Verfahren nach Anspruch 3, in dem das Cycloalken ein 5- bis 7-gliedriger olefinischer Ring oder ein 12-gliedriger olefinischer Ring ist.

5. Verfahren nach Anspruch 4, in dem das Cycloalken ein 5-gliedriger olefinischer Ring ist.

6. Verfahren nach Anspruch 1, in dem der allylartige Kohlensäureester ein Niederalkylester der Allylkohlensäure, Crotylkohlensäure oder Methallylkohlensäure ist.

7. Verfahren nach Anspruch 1, in dem der allylartige Kohlensäureester in einer Menge von 0,8 bis 5 Mol pro

Mol des Alkenylesters eingesetzt wird.

8. Verfahren nach Anspruch 7, in dem der allylartige Carbonsäureester in einer Menge von 1 bis 5 Mol pro Mol des Alkenylesters eingesetzt wird.

9. Verfahren nach Anspruch 1, in dem die Verbindung eines Platinmetalls als Katalysator eine Verbindung eines Platinmetalls oder ein Katalysator ist, der aus einer Verbindung eines Platinmetalls und einem Liganden zusammengesetzt ist.

10. Verfahren nach Anspruch 9, in dem die Verbindung eines Platinmetalls ein Salz oder ein Komplex eines Platinmetalls gewählt aus Palladium, Platin, Rhodium, Iridium oder Ruthenium ist.

11. Verfahren nach Anspruch 10, in dem das Platinmetall Palladium ist.

12. Verfahren nach Anspruch 11, in dem das Palladium als null-wertige Olefinkomplexe oder als zweiwertige organische Verbindung vorliegt.

13. Verfahren nach Anspruch 9, in dem der Ligand eine einzähnige oder mehrzähnige Elektronendonorverbindung ist, die als Ligandenatom ein Element der Gruppe V des Periodensystems aufweist.

14. Verfahren nach Anspruch 13, in dem das Ligandenatom aus Stickstoff, Phosphor, Arsen und Antimon gewählt ist.

15. Verfahren nach Anspruch 14, in dem der Ligand eine stickstoffhaltige Verbindung oder eine phosphorhaltige Verbindung ist.

16. Verfahren nach Anspruch 9, in dem der Ligand in einer Menge von 2,5 Mol oder weniger pro Mol der Verbindung eines Platinmetalls eingesetzt wird.

17. Verfahren nach Anspruch 16, in dem der Ligand in einer Menge von etwa 2 Mol oder weniger pro Mol der Verbindung eines Platinmetalls eingesetzt wird.

18. Verfahren nach Anspruch 1, in dem die Verbindung eines Platinmetalls als Katalysator in einer Menge von 0,01 bis 10 Mol pro 100 Mol des Alkenylesters eingesetzt wird.

19. Verfahren nach Anspruch 1, in dem die katalytische Umsetzung in Gegenwart eines Verdünnungsmittels durchgeführt wird.

20. Verfahren nach Anspruch 19, in dem das Verdünnungsmittel aus Nitrilen, Amiden, Ethern, Ketonen, Estern, Alkoholen, Sulfoxiden und Kohlenwasserstoffen gewählt ist.

21. Verfahren nach Anspruch 19, in dem das Verdünnungsmittel ein aprotisches,polares Lösungsmittel ist.

22. Verfahren nach Anspruch 1, in dem das Verdünnungsmittel in einer solchen Menge verwendet wird, daß die Gesamtkonzentration des Alkenylesters und des allylartigen Carbonylesters 1 bis 50 Gew.-% beträgt.

23. Verfahren nach Anspruch 1, in dem in der katalytischen Umsetzung die Umsetzungstemperatur 50°C oder höher ist und die Umsetzungszeit 10 Minuten bis 20 Stunden beträgt.

24. Verfahren nach Anspruch 1, in dem die phosphorhaltige Verbindung eine einzähnige oder mehrzähnige phosphorhal tige Elektronendonorverbindung ist.

25. Verfahren nach Anspruch 24, in dem die phosphorhaltige Verbindung ein Phosphin oder ein Phosphit ist.

26. Verfahren nach Anspruch 25, in dem das Phosphin ein Trialkylphosphin oder Triphenylphosphin ist.

27. Verfahren nach Anspruch 1, in dem in der Umsetzung unter Erhitzen die Umsetzungstemperatur 50°C oder höher und die Umsetzungszeit 30 Minuten bis 10 Stunden beträgt.

## Revendications

1. Procédé de préparation d'un composé carbonylé α,β-insaturé représenté par la formule (II) :

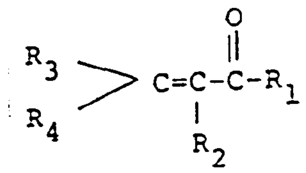

(II)

dans laquelle $R_1$, $R_2$, $R_3$ et $R_4$ représentent indépendamment des atomes d'hydrogène ou des résidus hydrocarbonés, et $R_1$, $R_2$, $R_3$ et $R_4$ peuvent être linéaires ou former un ou plusieurs cycles lorsqu'ils sont pris ensembles dans des combinaisons arbitraires,
caractérisé en ce qu'il comprend une réaction catalytique entre un ester alcènylique représenté par la formule générale (I):

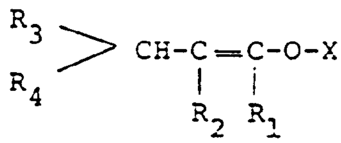

(I)

dans laquelle $R_1$, $R_2$, $R_3$ et $R_4$ représentent les mêmes termes que ceux définis ci-dessus, et X un groupe acyle,
et un ester carbonique ayant au moins un résidu allyle ou un résidu de même type et représenté par la formule générale (III) :

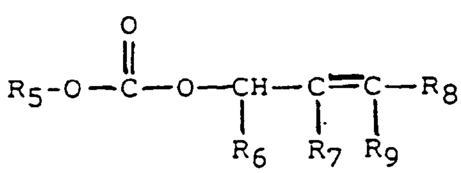

dans laquelle $R_5$ représente un résidu hydrocarboné et $R_6$, $R_7$, $R_8$ et $R_9$ indépendamment des atomes d'hydrogène ou des résidus hydrocarbonés, en présence d'un catalyseur composé d'un métal du groupe du platine, puis l'addition d'un composé contenant du phosphore, suivie de chauffage, et dans lequel la quantité totale de composé contenant du phosphore, utilisée dans la réaction catalytique et dans la réaction sous chauffage, est d'au moins 3 moles par mole de catalyseur composé d'un métal du groupe du platine.

2. Procédé conforme à la revendication 1, caractérisé en ce que $R_1$,$R_2$,$R_3$ et $R_4$, q'ils soient identiques ou différents, sont des atomes d'hydrogène, des groupes alkyle, alcoylène ou aryle.

3. Procédé conforme à la revendication 1, caractérisé en ce que l'ester alcènylique est un ester cycloalcènylique.

4. Procédé conforme à la revendication 3, caractérisé en ce que le cycloalcène est une oléfine cyclique de 5 à 7 chaînons ou une oléfine cyclique à 12 chaînons.

5. Procédé conforme à la revendication 4, caractérisé en ce que le cycloalcène est une oléfine cyclique à 5 chaînons.

6. Procédé conforme à la revendication 1, caractérisé en ce que l'ester de type allyl-carbonique est un ester alkylique inférieur d'allylcarbonate, de crotylcarbonate ou de méthallylcarbonate.

7. Procédé conforme à la revendication 1, caractérisé en ce que l'ester carboxylique de type allyle est utilisé en quantités comprises entre 0,8 et 5 moles par mole d'ester alcènylique.

8. Procédé conforme à la revendication 7, caractérisé en ce que l'ester de type allyl-carbonique est utilisé à raison de 1 à 5 moles par mole d'ester alcènylique.

9. Procédé conforme à la revendication 1, caractérisé en ce que le catalyseur composé d'un métal du groupe du platine est un composé d'un métal du groupe du platine ou un métal du groupe du platine et un ligand.

10. Procédé conforme à la revendication 9, caractérisé en ce que le composé d'un métal du groupe du platine est un sel ou un complexe d'un métal du groupe du platine choisi parmi le palladium, le platine, le rhodium, l'iridium ou le ruthénium.

11. Procédé conforme à la revendication 10, caractérisé en ce que le métal du groupe du platine est le palladium.

12. Procédé conforme à la revendication 11, caractérisé en ce que le palladium est sous forme d'un complexe zérovalent d'une oléfine ou d'un composé organique divalent.

13. Procédé conforme à la revendication 9, caractérisé en ce que le ligand est un composé donneur d'électrons mono- ou multidenté ayant comme atome ligand un élément du groupe V du tableau périodique.

14. Procédé conforme à la revendication 13, caractérisé en ce que l'atome ligand est choisi parmi l'azote, le posphore, l'arsenic, et l'antimoine.

15. Procédé conforme à la revendication 14, caractérisé en ce que le ligand est un composé contenant de l'azote ou du phosphore.

16. Procédé conforme à la revendication 9, caractérisé en ce que l'on utilise une quantité de ligand inférieure ou égale à 2,5 moles par mole de composé d'un métal du groupe du platine.

17. Procédé conforme à la revendication 16, caractérisé en ce que l'on utilise une quantité de ligand inférieure ou égale à environ 2 moles par mole de composé d'un métal du groupe du platine.

18. Procédé conforme à la revendication 1, caractérisé en ce que le catalyseur composé d'un métal du groupe du platine est utilisé à raison de 0,01 à 10 moles par 100 moles d'ester alcènylique.

19. Procédé conforme à la revendication 1, caractérisé en ce que la réaction est effectuée en présence d'un diluant.

20. Procédé conforme à la revendication 19, caractérisé en ce que l'on choisit le diluant parmi les nitriles, les amides, les éthers, les cétones, les esters, les alcools, les sulfoxydes et les hydrocarbures.

21. Procédé conforme à la revendication 19, caractérisé en ce que le diluant est un solvant polaire aprotique.

22. Procédé conforme à la revendication 1, caractérisé en ce que le diluant est utilisé en quantités telles que la concentration totale des esters alcènyliques et des esters de type allyl-carbonique soit comprise entre 1 et 50% en poids.

23. Procédé conforme à la revendication 1, caractérisé en ce que la température de la réaction catalytique est de 50 °C ou plus, et la durée de la réaction est comprise entre 10 minutes et 20 heures.

24. Procédé conforme à la revendication 1, caractérisé en ce que le composé contenant du phosphore est un composé donneur d'électrons mono- ou multidenté contenant du phosphore.

25. Procédé conforme à la revendication 24, caractérisé en ce que le composé contenant du phosphore est une phosphine ou un phosphite.

26. Procédé conforme à la revendication 25, caractérisé en ce que la phosphine est une trialkylphosphine ou une triphénylphosphine.

27. Procédé conforme à la revendication 1, caractérisé en ce que la température de réaction sous chauffage est de 50 °C ou plus et la durée de réaction est de 30 minutes à 10 heures.